# EUROPEAN PATENT APPLICATION

(11) **EP 4 389 164 A1**
(43) Date of publication of application: **26.06.2024**
(21) Application number: 22214867.8
(22) Date of filing: 20.12.2022
(51) Int. Cl.: A61M 1/06

(54) **MILK EXTRACTION SYSTEM**

(71) Applicant: Koninklijke Philips N.V., 5656 AG Eindhoven (NL)
(72) Inventor: VAN BENTUM, Jesper William, Eindhoven (NL); LIPSCH, Job, Eindhoven (NL); VAN DER KOOI, Johannes Tseard, Eindhoven (NL); PALERO, Jonathan Alambra, Eindhoven (NL); VAN DEN BIJGAART, Adrianus Wilhelmus Dionisius Maria, Eindhoven (NL); WIJNOLTZ, Anna Louise, Eindhoven (NL)
(74) Representative: Philips Intellectual Property & Standards

(57) **Abstract**

A milk extraction system comprises a body for placing over a nipple of a user, wherein the body comprises a first portion which is configured to form a milk extraction chamber over the nipple and a second portion which forms a pressure reduction chamber. The volume of the pressure reduction chamber can be reduced by manually deforming the second portion thereby to result in a reduced pressure within the pressure reduction chamber. Using one-way valves, the pressure in the pressure reduction chamber is substantially equilibrated with the milk extraction chamber, and re-pumping is also enabled, so that the milk extraction chamber can be re-pressurized by a series of pumping operations.

## Description

### FIELD OF THE INVENTION

This invention relates to milk extraction systems, in particular forming part of a milk collection systems, for collecting milk from a lactating mother.

### BACKGROUND OF THE INVENTION

The best source of nutrition for a baby is human milk given by a breastfeeding mother. The world health organization recommends to breast feed for at least for the first six months of life, followed by continued breastfeeding with appropriate complementary foods for up to 2 years and beyond.

However, typically mothers often go back to work after already several weeks or months. To provide the best nutrition to their babies, mothers express milk using a breast pump. The expressed milk can be stored and given to the baby at a later stage and/or by somebody else.

Breast pumps typically unit make use of a drive arrangement for controlling an expression function. The drive arrangement has a pump for generating a vacuum in a vacuum chamber of the breast pump. A flexible membrane known as the diaphragm is located in the vacuum chamber. The pump is controlled to apply a vacuum to the diaphragm so that it deforms to create a vacuum in a breast receiving funnel, when it is sealed to the breast. This in turn applies a vacuum to the breast which enables milk to be expressed.

The vacuum is applied to the breast at intervals. That is, a pressure differential is applied on a cyclic basis. There are also wearable breast pumps, which are for example for mounting within a feeding bra.

Recently, there is a trend towards wearable passive milk collectors that collect leaking milk. The wearable passive milk collector can only be used with the baby present and feeding on the other breast.

When the baby starts to suckle at a breast, nipple stimulation causes the milk release (MER) at both breasts. The milk collector then collects milk from the other breast through a passive constant negative pressure (vacuum). The milk collector is for example formed as a compressible milk container which can be pressed inwards for generating a base vacuum for enhancing the milk extraction. It can also provide a holding pressure against the breast, although this may be achieved by separate means.

When the user compresses the milk collector and places the collector on her breast to generate under-pressure, it is important that the right amount of under-pressure is generated to reach a MER and more importantly, to extract the milk from her breast after the MER.

After reaching a MER, the passive negative pressure created when the milk collection system is initially deployed will slowly decrease due to the milk entering the collector. Eventually, the milk collector will proceed to reach the ambient atmospheric pressure level if the user does not intervene. The loss of under-pressure means the milk extraction will be less effective, and there is also the possibility of the filled milk collector falling off the breast. Therefore, it is sometimes necessary to generate more negative pressure, by performing a re-pumping action, so the user can continue collecting milk.

A large-volume milk collector could make re-pumping unnecessary because it can generate sufficient vacuum in one application, but this makes the milk collector less discreet and compact for the user and certainly not in a wearable form. A smaller volume however requires a re-pumping operation.

Re-pumping with current available milk collectors is not at all practical. To generate more under-pressure while collecting milk, users must compress the milk collector to squeeze out the remaining air in the collector. But this also allows the milk to be squeezed out of the collector, resulting in spillage and user frustration. An electric vacuum pump could be used to maintain the negative pressure but this results in a more complex, bulky device and is eventually more costly.

Finally, while wearing a wearable milk collector, it is difficult for the user to see when the milk collector is approaching atmospheric pressure. The surface to visually judge whether the collector has enough negative pressure is often located at the front or bottom of the device and sometimes covert by clothing or a bra.

There is therefore a need for a milk collector that can be re-pumped by the user to increase the under-pressure without the risk of leakage. Another benefit would be the ability to visually ascertain the level of under-pressure, so that the user knows when re-pumping is needed.

### SUMMARY OF THE INVENTION

The invention is defined by the claims.

According to examples in accordance with an aspect of the invention, there is provided a milk extraction system comprising:
a body for placing over a nipple of a user, wherein the body comprises:
   a first portion which is configured to form a milk extraction chamber over the nipple; and
   a second portion which forms a pressure reduction chamber;
a first one-way valve between the milk extraction chamber and the pressure reduction chamber, to enable air to flow from the milk extraction chamber to the pressure reduction chamber; and
a second one-way valve between the pressure reduction chamber and ambient surroundings around the system to enable air to be released from the pressure reduction chamber to the ambient surroundings,
wherein the second portion comprises at a least a part that is elastically deformable such that the volume of the pressure reduction chamber can be reduced by manually deforming the second portion thereby to result in a reduced pressure within the pressure reduction chamber and the milk extraction chamber.

When the second portion is deformed to reduce the volume of the pressure reduction chamber, the second valve allows air to escape. The elasticity of the second portion tries to return the pressure reduction chamber to its original shape, but the second valve is then closed. The force resulting from the elasticity of the second portion causes a reduction in pressure. This pressure reduction is substantially equilibrated with the milk extraction chamber because the first valve is open by the prevailing pressure difference (it is "substantially" equilibrated in that a pressure drop will still exist across the first valve even through it is open). Thus, activation of the pressure reduction chamber (e.g. squeezing or pushing it to reduce its volume) is used to reduce the pressure in the milk extraction chamber and hence enable the system to remain fitted to the breast/nipple and stimulating the MER. However, by avoiding the need to expel air from the milk extraction chamber around the breast (and instead expelling air into the pressure reduction chamber) the risk of milk spillage is reduced.

The milk extraction system is part of a milk collection system for passive collection of leaking milk from the breast of a breastfeeding mother. The milk collection system is passive in the sense that there is no generation of a complex cyclic waveform. An under-pressure level is used, but it is generated manually, so there is no need for an electric or mechanical pump, and hence no need for a controller, or any actuators.

It is noted that the second portion may be elastically deformable by virtue of the material of the housing (e.g. rubber) but there may be separate elements to provide the desired elasticity, such as springs.

The first portion is for example also comprises at least a part that is elastically deformable such that the volume of the milk extraction chamber can be reduced by manually deforming that part of the first portion thereby to create a reduced pressure within the milk extraction chamber.

The manual deformation of the first portion of the housing is for example used for initial fitting of the system, before it has collected any milk. The pressure reduction chamber is then used for re-application of a reduced pressure. The milk extraction chamber may thus be considered to be used for an initial pumping function and the pressure reduction chamber may be considered to be used for a re-pumping function.

It is noted that, similar to the second portion, the first portion may be elastically deformable by virtue of the material of the corresponding portion of the body (e.g. rubber) but there may be separate elements to provide the desired elasticity, such as springs.

The first and second portions are for example formed of silicone.

The first and second one-way valves for example each comprise at least one duckbill valve. This is a simple valve structure.

The duckbill valves are for example formed as integral parts of the body. This provides simple manufacturing. In particular, the duckbill valves and said elastically deformable part of the second portion may be formed as a single molded item.

The pressure reduction chamber is for example deformable between a rest state and a deformed state, wherein the pressure reduction chamber is configured to retain the deformed state when an under-pressure level within the pressure reduction chamber exceeds a threshold under-pressure.

A pressure with a range of under-pressure levels is desired, sufficient for promoting milk extraction but not so great as to cause bruising. During re-re-pumping, this feature enables the user to have an indication of when the under-pressure level has reached a threshold. In this way, the user can re-pump until the pressure reduction chamber no longer returns back to its rest state, indicating that a desired under-pressure level has been reached. The pressure level at which the pressure reduction chamber no longer returns to its rest state is a function of the elastic properties of the pressure reduction chamber and the milk extraction chamber.

The milk extraction system may comprising an outer frame over the body. This can prevent snagging of clothes or a bra on the housing.

The invention also provides a milk collection system comprising the milk extraction system defined above, wherein the milk extraction chamber forms or is connected to a milk collection chamber.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known milk collection system;
Fig. 2 shows a first example of a milk expression system of the invention;
Fig. 3 shows the stages of use of the system of Fig. 2; and
Fig. 4 shows a second example of a milk expression system of the invention and is used to explain an additional optional feature.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a milk extraction system comprising a body for placing over a nipple of a user, wherein the body comprises a first portion which is configured to form a milk extraction chamber over the nipple and a second portion which forms a pressure reduction chamber. The volume of the pressure reduction chamber can be reduced by manually deforming the second portion thereby to result in a reduced pressure within the pressure reduction chamber. Using one-way valves, the pressure in the pressure reduction chamber is substantially equilibrated with the milk extraction chamber, and re-pumping is also enabled, so that the milk extraction chamber can be re-pressurized by a series of pumping operations.

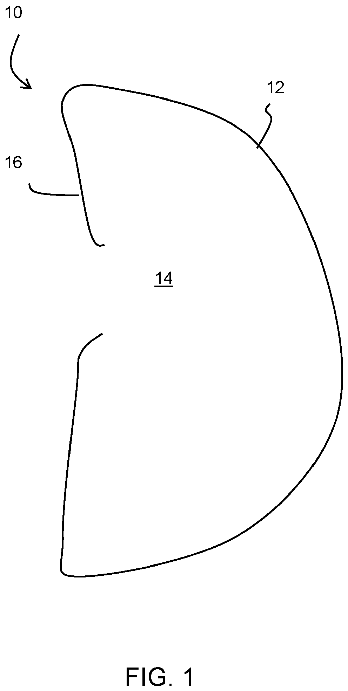

Fig. 1 shows a known milk collection system, comprising a housing 12 for placing over a nipple of a user thereby to form a milk extraction chamber 14 over the breast.

The primary purpose of the milk extraction chamber 14 is to apply an under-pressure (i.e. a pressure below atmospheric pressure) to the nipple of the user. This under-pressure promotes milk extraction, and is for example used once the milk ejection reflex has been stimulated on the other breast. As a secondary purpose, the pressure is used to hold the milk extraction chamber on the nipple, although other mechanisms may perform this purpose.

In the example shown, the milk extraction chamber is a milk collection chamber, i.e. the milk is extracted directly into a milk collection vessel. However, a milk collection vessel and the milk extraction chamber may be separate devices, with a fluid connection between the milk extraction chamber and the milk collection vessel. The separate milk extraction chamber and milk collection vessel may be at the same pressure, or else there may be a valve between them so that only the milk extraction chamber is operated at a reduced pressure.

In this example (wherein the housing forms a milk collection vessel) the housing has a lip 16 which is deformed over the breast to create a seal with the breast tissue when the housing is positioned over the breast.

The housing is elastically deformable, so that it has a rest state (as shown). By manually deforming the housing, e.g. by squeezing the housing 12, the volume of the milk extraction chamber 14 is reduced. Air is pushed past the lip 16 but cannot flow the other way. Thus, the air volume is reduced, and when the housing 12 tries to return to its original shape due to its elasticity, an under-pressure is created within the milk extraction chamber defined by the housing 12.

The milk collection system is passive, with no active (e.g. electrical) generation of a complex cyclic waveform.

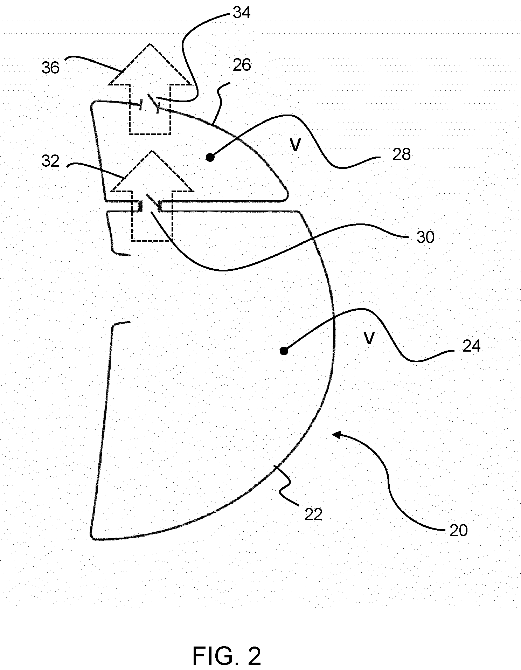

Fig. 2 shows a milk extraction system 20 in accordance with an example of the invention, comprising a body for placing over a nipple of a user. The body comprises a first portion 22 which is configured to form a milk extraction chamber 24 over the nipple and a second portion 26 which forms a pressure reduction chamber 28.

A first one-way valve 30 is between the milk extraction chamber 24 and the pressure reduction chamber 28, to enable air to flow from the milk extraction chamber to the pressure reduction chamber as shown by arrow 32. A second one-way valve 34 is between the pressure reduction chamber 28 and ambient surroundings around the system to enable air to be released from the pressure reduction chamber to the ambient surroundings as shown by arrow 36.

The second portion 26 of the body, and hence the pressure reduction chamber 28, is elastically deformable such that the volume of the pressure reduction chamber 28 can be reduced by manually deforming the second portion thereby to result in a reduced pressure within the pressure reduction chamber. This reduced pressure results in a reduced pressure in the milk extraction chamber as a result of the open valve 30 (which means the two pressures may be considered to be substantially equilibrated).

In the same way as the milk collection chamber of Fig. 1, the elasticity of the second portion 26 tries to return the pressure reduction chamber to its original shape, but the second valve 34 is then closed by the pressure differential across the valve (i.e. by the higher pressure of the ambient surroundings). The force resulting from the elasticity of the second portion causes a reduction in pressure. This reduction opens the first valve 30 so that the reduced pressure is substantially equilibrated with the milk extraction chamber 24. Thus, activation of the pressure reduction chamber (e.g. squeezing or pushing it to reduce its volume) is used to reduce the pressure in the milk extraction chamber without expelling air from the milk extraction chamber 24 around the breast. Thus, the risk of milk spillage is reduced.

The first portion 22 is preferably also elastically deformable such that the volume of the milk extraction chamber can be reduced by manually deforming the first portion thereby to create a reduced pressure within the milk extraction chamber.

The milk extraction chamber is typically larger than the pressure reduction chamber. The manual manipulation of the milk extraction chamber is used initially to fit the system to the breast or nipple, and the manual manipulation of the pressure reduction chamber is used to re-apply a reduced vacuum to the milk extraction chamber subsequently.

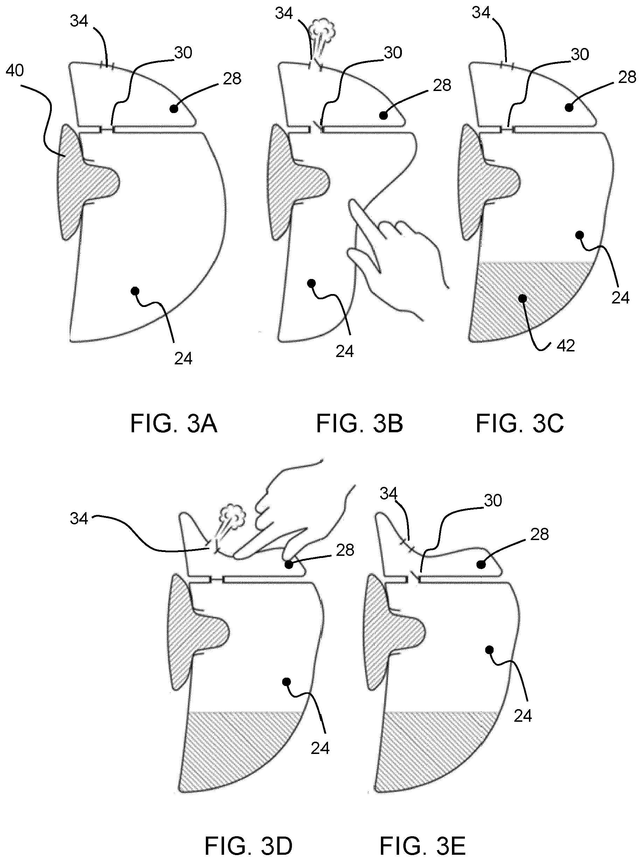

Fig. 3 shows how the milk extraction system is used.

Fig. 3A shows an initial rest state of the system immediately after fitting over the nipple 40 of to the user.

Fig. 3B shows the initial application of an under-pressure. Manual actuation of the milk extraction chamber 24 results in a reduction in volume. Air is expelled through the first one-way valve 30 to the pressure reduction chamber 28 and from the pressure reduction chamber 28 through the second one-way valve 34 to the ambient surroundings. Once the manual force is removed, the milk extraction chamber tries to return to its original shape. This creates suction, which closes the two valves.

When the milk extraction chamber has returned to its rest shape, there is then an under-pressure within both chambers.

Fig. 3C shows milk 42 having been collected. This results in a reduction in the level of under-pressure.

Fig. 3D shows a re-pressurization by manual actuation of the pressure reduction chamber. Air is expelled from the pressure reduction chamber 28 to the ambient surroundings by the open second one-way valve 34, whereas the first one-way valve 30 remains closed. Once the manual force is removed as shown in Fig. 3E, the pressure reduction chamber tries to return to its shape creating an under-pressure which opens the first one-way valve 30 but closes the second one-way valve 34.

Thus, during the re-pumping, the first and second one-way valves are operated with an alternating sequence.

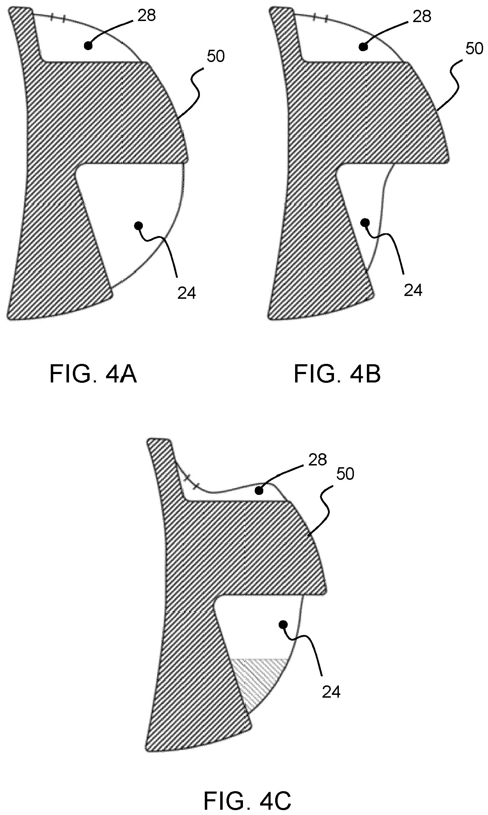

Fig. 3E shows the pressure reduction chamber in its deformed state even after removal of the manual force. This will become clear from the discussion below with reference to Fig. 4.

Fig. 4 shows a second example of milk extraction system and is also used to explain another optional feature.

An outer frame 50 is provided over the housing. The frame provides a hard shell, which prevents the user's bra or clothing from interfering with the milk collector's pressure profile.

Fig. 4 is used to show that the pressure reduction chamber 28 can also act as a visual indicator for the user. For example, when a milk collection system is worn underneath clothing or a bra, the user is still able to see if the collector has a sufficient under-pressure level.

Fig. 4A shows the shape of the overall system without any negative pressure. Fig. 4B shows the shape of the system with an under-pressure in the milk extraction chamber 24 and Fig. 4C shows the shape of the system with an under-pressure in the pressure reduction chamber 28, which acts as a visual indicator on level of under-pressure.

In particular, the pressure reduction chamber is deformable between a rest state (Fig. 4A) and a deformed state (Fig. 4C). The pressure reduction chamber retains the deformed state when the under-pressure level within the pressure reduction chamber (and hence also a corresponding under-pressure level in the milk extraction chamber) exceeds a threshold under-pressure. This is the condition shown in Fig. 4C. The pressure reduction chamber is more elastic than the milk extraction chamber so it preferentially deforms in response to a prevailing under-pressure level. In other words, the system will adopt the configuration of Fig. 4C in preference to the configuration of Fig. 4B. Furthermore, when the pressure reduction chamber is retained in the deformed (collapsed) state, this indicates that the prevailing under-pressure level is greater than a threshold level (i.e. the pressure is below a threshold value). By having the pressure reduction chamber visible in use (e.g. from above), its collapsed state is indicative of a suitable pressure.

Furthermore, the re-application of pressure comprises a sequence of pumping actions (e.g. multiple cycles between Figs. 3C and 3D before Fig. 3E is reached). When the pressure reduction chamber no longer returns to its rest shape, this is an indication that a suitable under-pressure level has been reached.

The first and second portions of the body are for example formed of silicone. The first and second one-way valves for example each comprise at least one duckbill valve, and they may be formed as integral molded parts of the body.

The body (and both portions and valves defined by it) may be a single molded item, or the chambers may be separately formed and connected. In other examples, the valves may be inserts which are applied to the chambers instead of being integrally formed with them.

The examples above are based on deformable chambers which define the external appearance of the milk collection system. However, in other examples, the deformable chambers may be covered by other parts which are then actuated by the user to induce the deformation of the chambers. For example, a rotary knob may be provided which operates a plunger. The chambers themselves may be twisted, e.g. on a threaded shaft, to induce the change in volume.

Thus, the user does not necessarily interact directly with the chambers, but the manual deformation may instead be indirect, in that the manual interaction of the user is with another part of the pressure control arrangement. There is still however manual deformation of the housing in that no independently powered actuator is used.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A milk extraction system (20) comprising:
a body for placing over a nipple of a user, wherein the body comprises:
a first portion (22) which is configured to form a milk extraction chamber (24) over the nipple; and
a second portion (26) which forms a pressure reduction chamber (28);
a first one-way valve (30) between the milk extraction chamber and the pressure reduction chamber, to enable air to flow from the milk extraction chamber to the pressure reduction chamber; and
a second one-way valve (34) between the pressure reduction chamber and ambient surroundings around the system to enable air to be released from the pressure reduction chamber to the ambient surroundings,
wherein the second portion comprises at a least a part that is elastically deformable such that the volume of the pressure reduction chamber (28) can be reduced by manually deforming the second portion thereby to result in a reduced pressure within the pressure reduction chamber (28) and the milk extraction chamber (24).

2. The milk extraction system of claim 1, wherein the first portion (22) also comprises at least a part that is elastically deformable such that the volume of the milk extraction chamber can be reduced by manually deforming the first portion thereby to create a reduced pressure within the milk extraction chamber.

3. The milk extraction system of claim 1 or 2, wherein the first and second portions (22, 26) are formed of silicone.

4. The milk extraction system of any one of claims 1 to 3, wherein the first and second one-way valves (30, 34) each comprise at least one duckbill valve.

5. The milk extraction system of claim 4, wherein the duckbill valves are formed as integral parts of the body.

6. The milk extraction system of any one of claims 1 to 5, wherein the pressure reduction chamber (28) is deformable between a rest state and a deformed state, wherein the pressure reduction chamber (28) is configured to retain the deformed state when an under-pressure level within the pressure reduction chamber (28) exceeds a threshold under-pressure.

7. The milk extraction system of any one of claims 1 to 6, comprising an outer frame (50) over the body.

8. A milk collection system comprising the milk extraction system of any one of claims 1 to 7, wherein the milk extraction chamber (24) forms or is connected to a milk collection chamber.
